# EUROPEAN PATENT SPECIFICATION

(11) **EP 3 381 424 B1**
(45) Date of publication and mention of the grant of the patent: **03.06.2020**
(21) Application number: 16888010.2
(22) Date of filing: 18.02.2016
(51) Int. Cl.: A61F 13/15, A61F 13/494

(54) **ABSORBENT ARTICLE**
SAUGFÄHIGER ARTIKEL
ARTICLE ABSORBANT

(30) Priority: 29.01.2016 JP 2016016492
(43) Date of publication of application: 03.10.2018
(73) Proprietor: Unicharm Corporation, Ehime 799-0111 (JP)
(72) Inventor: KAWAKAMI, Yusuke, Kanonji-shi Kagawa 769-1602 (JP); SHIMOTSU, Maiko, Kanonji-shi Kagawa 769-1602 (JP); NAGAI, Takahito, Kanonji-shi Kagawa 769-1602 (JP); SONODA, Junko, Kanonji-shi Kagawa 769-1602 (JP)
(74) Representative: Dolleymores
(86) International application number: PCT/JP2016/054680
(87) International publication number: WO 2017/130424

(56) References cited:
- WO-A1-2015/182205
- JP-A- 2002 078 734
- JP-A- 2005 007 076
- JP-A- 2005 218 876
- JP-A- 2011 120 764
- JP-A- 2014 108 270

## Description

### [Technical Field]

The present disclosure relates to an absorbent article.

### [Background Art]

Conventionally, pull-on disposable diapers have been used. Such a common pull-on disposable diaper in an unfolded state has a longitudinal direction and a width direction, and includes an absorbent main body provided with an absorbent core that absorbs excrement. Then, leak-proof wall portions that prevent excrement absorbed by the absorbent core from leaking outside the diaper are provided on both sides in the width direction of the absorbent body along the circumferences of a wearer's legs. For example, Patent Literature 1 discloses a disposable diaper in which side flaps are provided on both sides of a liquid absorbent panel (absorbent main body), and elastic members such as elastic strings provided on the free end side of the side flaps contract to form gathers (leak-proof wall portions).

### [Citation List]

### [Patent Literature]

[PTL 1] Japanese Patent Application Publication No.2009-61052
WO 2015/182205 A1 discloses an open-type absorbent article having leak-proof walls.

### [Summary of Invention]

### [Technical Problem]

When the disposable diaper is worn, the absorbent core absorbs excrement thereby increasing its weight, and the absorbent main body droops downward under its own weight, so that a space may be produced at a wearer's crotch part (groin part) and excrement may leak outside from leg-circumferential openings. In such an occasion, in the conventional diaper as described in PTL 1, leak-proof walls deviate in position in association with drooping of the absorbent main body, and thus it is difficult to sufficiently suppress creation of such a space. Further, tightening of elastic members provided to the leak-proof walls so as not to create a space may bring discomfort to a wearer.

The present disclosure has been made in view of the conventional issues as described above, and an aspect thereof is to suppress formation of a space in a crotch part while restraining discomfort from being brought to a wearer, in an absorbent article including leak-proof walls.

### [Solution to Problem]

The present invention provides the absorbent article of independent claim 1. The dependent claims specify preferred but optional features.

A primary aspect of the disclosure is an absorbent article, comprising: an absorbent main body including an absorbent core having a longitudinal direction and a lateral direction intersecting each other; and leak-proof walls in a pair respectively provided on both sides in the lateral direction of the absorbent main body, the leak-proof walls including elastic members that are stretchable in the longitudinal direction, the absorbent core including a concave portion that is concave inward in the lateral direction in the central portion in the longitudinal direction, a dimension in the lateral direction of the concave portion provided in the absorbent core being smaller than a dimension in the lateral direction of both ends in the longitudinal direction of the absorbent core, the leak-proof walls each including a joining part where portions of a surface of each of the leak-proof walls are joined to each other on one side in a thickness direction, the leak-proof walls each including a non-joining part in at least one of a region on one end side and a region on another end side in the longitudinal direction with respect to the joining part, the non-joining part being where portions of a surface of each of the leak-proof walls are not joined to each other on the one side in a thickness direction, the joining part provided in each of the leak-proof walls in a pair not overlapping the absorbent core in the thickness direction, a distance between the joining parts provided to the leak-proof walls in a pair being greater in the lateral direction than a minimum dimension of the concave portion and smaller in the lateral direction than a dimension of both the ends in the longitudinal direction of the absorbent core.

Other features of the present disclosure will be made clear from the descriptions of the present specification with reference to the accompanying drawings.

### [Advantageous Effects of Invention]

According to the present disclosure, it is possible to suppress formation of a space in a crotch part while restraining discomfort from being brought to a wearer in an absorbent article including leak-proof walls.

### [Brief Description of the Drawings]

FIG. 1 is a plan view illustrating a diaper 1 when unfolded and stretched.
FIG. 2 is a schematic perspective view when a diaper 1 is viewed from the front.
FIG. 3A is a schematic cross sectional view taken along a line A-A of FIG. 1.
FIG. 3B is a schematic cross sectional view taken along a line B-B of FIG. 1A.
FIG. 3C is a schematic cross sectional view taken along a line C-C of FIG. 1A.
FIG. 4 is a plan view illustrating leak-proof walls 50 when unfolded and stretched.
FIG. 5 is a schematic enlarged cross sectional view illustrating a region D of FIG. 3C.
FIG. 6A is a diagram illustrating a state of wearing a diaper 100 (comparative example) before excretion.
FIG. 6B is a diagram illustrating a state of wearing a diaper 100 (comparative example) after excretion.
FIG. 7A is a diagram illustrating a state of wearing a diaper 1 before excretion.
FIG. 7B is a diagram illustrating a state of wearing a diaper 1 after excretion.
FIG. 8 is a schematic plan view illustrating an arrangement of a folded joining part 61.

### [Description of Embodiments]

At least following matter will become clear from the descriptions of the present specification with reference to the accompanying drawings.

An absorbent article, comprising: an absorbent main body including an absorbent core having a longitudinal direction and a lateral direction intersecting each other; and leak-proof walls in a pair respectively provided on both sides in the lateral direction of the absorbent main body, the leak-proof walls including elastic members that are stretchable in the longitudinal direction, the absorbent core including a concave portion that is concave inward in the lateral direction in the central portion in the longitudinal direction, the leak-proof walls each including a joining part where portions of a surface of each of the leak-proof walls are joined to each other on one side in a thickness direction, in at least a part of a region corresponding to the concave portion in the longitudinal direction, the leak-proof walls each including a non-joining part in at least one of a region on one end side and a region on another end side in the longitudinal direction with respect to the joining part, the non-joining part being where portions of a surface of each of the leak-proof walls are not joined to each other on the one side in a thickness direction.

According to such an absorbent article, a portion obtained such that portions of each of the leak-proof walls are joined to each other by the joining part contact a wearer's body in a flat manner, so that the position of the joining part is less likely to deviate. Thus, a space is less likely to be formed between the wearer's body and the leak-proof walls. Further, the portions of each of the leak-proof wall are joined to each other, thereby lowering the height of the leak-proof walls in the concave portion, so that the absorbent core is less likely to droop downward. Accordingly, discomfort is less likely to be brought to the wearer.

In such an absorbent article, it is preferable that the non-joining part is included in each of a region on one end side and a region on the other end side in the longitudinal direction with respect to the joining part in each of the leak-proof walls.

According to such an absorbent article, the non-joining parts having higher softness than the joining part are respectively formed outside the joining part on both sides in the longitudinal direction, so that the leak-proof walls become deformable, and the leak-proof walls are likely to fit along a body shape when the absorbent article is worn.

In such an absorbent article, the joining part does not overlap the absorbent core in the thickness direction.

According to such an absorbent article, when the absorbent article is worn, the joining part having higher stiffness is suppressed from being pressed against a body by the absorbent core, texture is less likely to deteriorate. Further, in the region, a problem such as drooping of the absorbent core is not likely to occur, and thus there is less necessity for forming the joining part. Accordingly, it is possible to shorten the length in the longitudinal direction of the joining part.

In such an absorbent article, it is preferable that an end in the longitudinal direction of a region where a stretching force of the elastic member acts is positioned outside in the longitudinal direction with respect to an end in the longitudinal direction of the joining part.

According to such an absorbent article, it is possible that a stretching force by virtue of the elastic member (leak-proof wall elastic member) can be exerted on all the region of the joining part in the longitudinal direction. Accordingly, a section at which portions of each of the leak-proof walls are joined by the joining part is easily formed in a flat manner, and the leak-proof walls fit in a flat manner to a wearer's body so as to cause less deviation in position of the leak-proof walls.

In such an absorbent article, it is preferable that the leak-proof walls each include a portion obtained by folding each of the leak-proof walls outward from inside in the lateral direction at a folding line along the longitudinal direction.

According to such an absorbent article, the portion obtained by folding each of the leak-proof walls results in a free end on the outer side in the lateral direction. Accordingly, when the absorbent article is worn, the leak-proof walls protrude outward in the lateral direction, and when a wearer inserts his/her legs into the leg openings of the absorbent article, legs' fingers are less likely to be caught by the leak-proof walls, thereby facilitating putting on.

In such an absorbent article, it is preferable that the portion obtained by folding each of the leak-proof walls outward from inside in the lateral direction includes a fold part obtained by joining folded portions of the leak-proof wall to each other with the joining part, and a single layer part obtained by not joining folded portions of the leak-proof wall, in an outside of the fold part in the lateral direction, and stiffness, of the leak-proof wall, in the fold part is greater than stiffness, of the leak-proof wall, in the single layer part.

According to such an absorbent article, when the absorbent article is worn, a fold part having higher stiffness is firmly pressed against a wearer's body while a surface shape is maintained. On the other hand, since the single layer part having lower stiffness is deformable, and thus is pressed against the wearer's skin side while forming a curved face along a body's projections and depressions. This can cause the leak-proof walls to come in surface contact with the wearer's body, thereby improving fit.

In such an absorbent article, it is preferable that the fold part includes a top face portion to contact a wearer's skin, and a lower face portion overlapping the top face portion on its lower side.

According to such an absorbent article, the elastic member provided to the lower face portion contracts, to push the lower face portion and the top face portion upward to the wearer's skin side at the same time, so that the region where the lower face portion and the top face portion overlap to be joined to each other easily comes in surface contact with the wearer's skin while maintaining its surface shape. This enhances fit of the absorbent article.

In such an absorbent article, it is preferable that a position of the elastic member that is provided to the lower face portion is same as or inside, in the lateral direction, a position of the joining part.

According to such an absorbent article, the elastic member is disposed below or inside the joining part in the lateral direction, and thus a stretching force by virtue of the elastic member is exerted in a wide range in the top face portion and the lower face portion through the joining part, thereby causing the region to come in surface contact with a wearer's skin. This enhances fit of the absorbent article.

In such an absorbent article, it is preferable that a top sheet is provided on a skin side of the absorbent body, the leak-proof walls each includes a lower face portion obtained by folding the leak-proof wall inward from outside in the lateral direction to overlap the top sheet on its skin side, and a top face portion obtained by folding the leak-proof wall outward from inside in the lateral direction to overlap the lower face portion on its skin side, and the top sheet and the lower face portion are joined in a region outside the non-joining part in the longitudinal direction as well as outside the joining part in the lateral direction, so that the lower face portion and the top face portion are joined to each other.

According to such an absorbent article, in an end part in the longitudinal direction of each of the leak-proof walls, the top face portion and the lower face portion are fixed to the skin-side surface of the top sheet, so that the outer end portions in the lateral direction are suppressed from curling. Further, a portion in which the top face portion and the lower face portion results in a pocket structure, excrement absorbed by the absorbent core is easily suppressed from leaking outside the fixed part from the absorbent core.

In such an absorbent article, it is preferable that a distance in the lateral direction between the joining part and the concave portion in a position deviating from a center in the longitudinal direction of the absorbent core by a predetermined distance is equal to or greater than a distance in the lateral direction between the joining part and the concave portion at a position deviating from a center in the longitudinal direction of the absorbent core to a back side by the predetermined distance.

According to such an absorbent article, a distance from the outer end portion in the lateral direction of the absorbent core (length of standing parts of leak-proof walls) to the portion at which the leak-proof wall contacts a wearer's skin is shortened on the back side, so that the absorbent core is less likely to droop. Further, the distance in the lateral direction of the absorbent core on the front side, and thus when putting on the absorbent article, the fingers of legs of the wearer are easily restrained from being caught by the side end portion of the absorbent core.

In such an absorbent article, it is preferable that a leg-circumference elastic member configured to stretch in the lateral direction is disposed on the back side in the longitudinal direction, the leg-circumference elastic member includes a linear part disposed along the lateral direction so as to overlap the absorbent core in the thickness direction in a central portion in the lateral direction, and curved parts disposed to be curved from both sides in the lateral direction of the linear part to outside in the lateral direction as well as to the back side in the longitudinal direction, and the leak-proof wall and the leg-circumference elastic member have a portion intersecting each other in the thickness direction.

According to such an absorbent article, the leak-proof walls are pulled obliquely upward by virtue of the stretching force of the curved parts of the leg-circumference elastic member, so that the leak-proof walls easily stand up when the absorbent article is worn. Further, the width of the absorbent main body is easily increased with the leak-proof walls being pulled. This enhances fit of leg circumferences when the absorbent article is worn.

In such an absorbent article, it is preferable that the joining part and the leg-circumference elastic member have a portion intersecting each other in the thickness direction.

According to such an absorbent article, the joining parts are pulled obliquely upward with respect to the absorbent article by virtue of a stretching force of the curved parts of the leg-circumference elastic members, so that a portion (fold part) obtained by joining by the joining part is further firmly pressed against the wearer's skin side so that a space is not easily created in the leg circumferential portions. This enhances fit in the leg circumferences when the absorbent article is worn.

In such an absorbent article, it is preferable that the rate of stretch in the linear part is greater than the rate of stretch in the curved parts in the leg-circumference elastic member.

According to such an absorbent article, in the central portion in the lateral direction, the rate of stretch of the linear part overlapping the absorbent core is lowered, thereby being able to suppress the absorbent core 11 from excessively contracting in the lateral direction. Further, the rate of stretch of the curved part to contact a wearer's buttocks when wearing the absorbent article is lowered, thereby strengthening the contractive force so that positional deviation can be less likely to be caused.

In such an absorbent article, it is preferable to include a front waist elastic member is disposed along the lateral direction on the front side in the longitudinal direction, the front waist elastic member being stretchable in the lateral direction; and a back waist elastic member is disposed along the lateral direction on the back side in the longitudinal direction, the back waist elastic member being stretchable in the lateral direction, and at least one of a region where stretchability is exerted by the front waist elastic member and a region where stretchability is exerted by the back waist elastic member intersects, in the thickness direction, a region where stretchability is exerted by the elastic members provided to the leak-proof walls.

In such an absorbent article, the elastic members provided to the leak-proof walls are supported by stretchability in the lateral direction of the front waist elastic member and/or the back waist elastic member, and thus the leak-proof walls and the absorbent main body are less likely to droop downward. Accordingly, even in the case where the absorbent core absorbs excrement, etc., thereby increasing its weight, the absorbent main body is suppressed from greatly drooping, so that discomfort is less likely to be brought to a wearer.

### ===Embodiments===

### <Basic configuration of diaper 1>

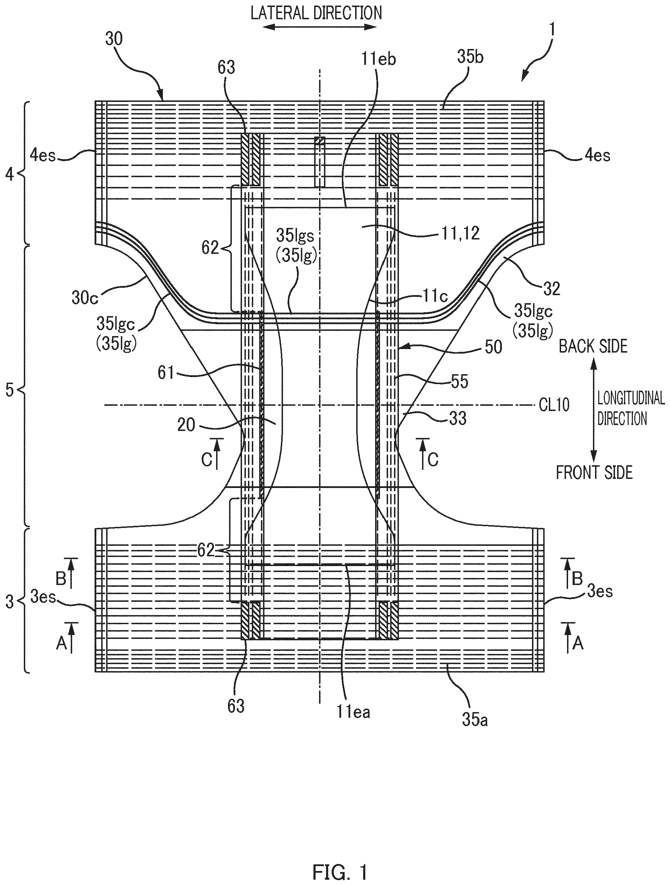

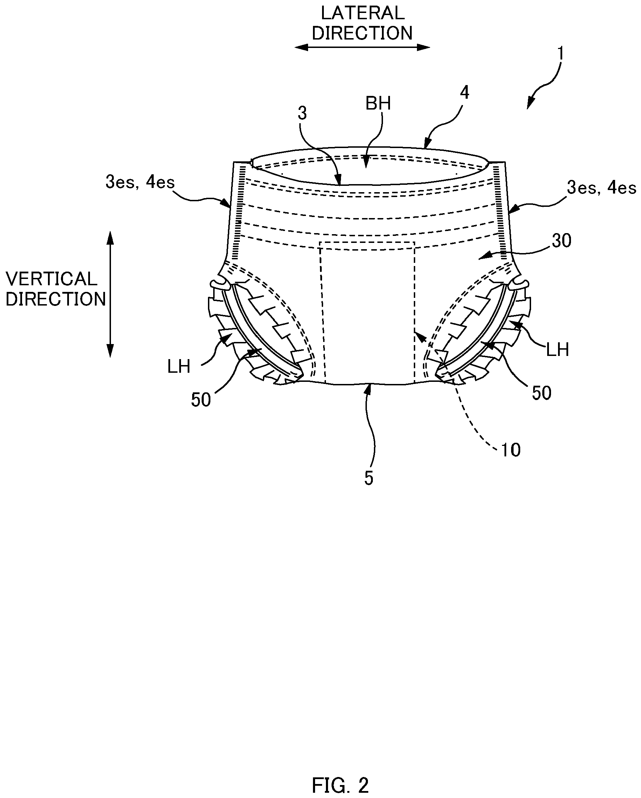

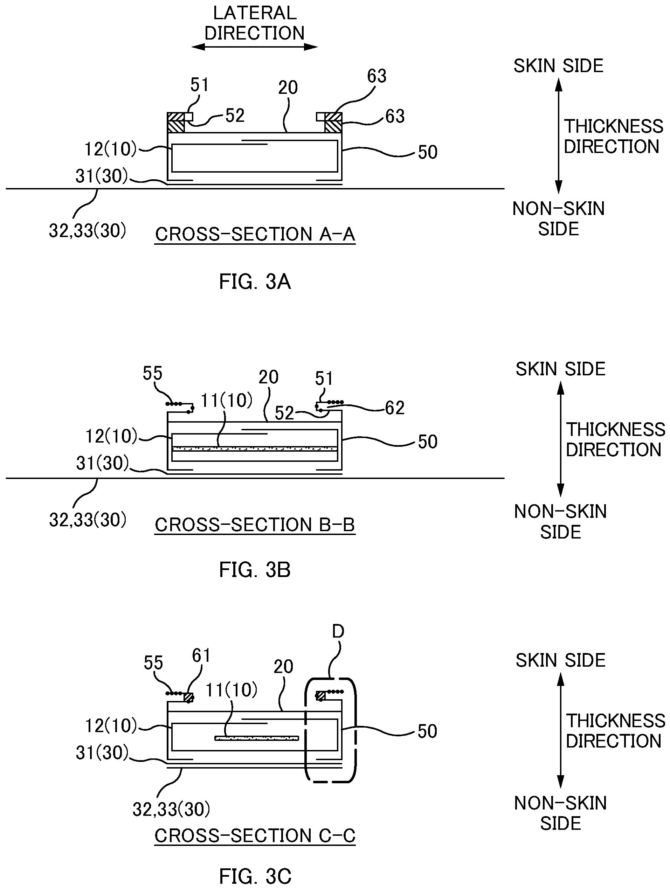

A basic configuration of a pull-on disposable diaper 1 (hereinafter, also referred to as the "diaper 1") will be described as one example of an absorbent article according to an embodiment of the present disclosure. FIG. 1 is a plan view illustrating the diaper 1 when unfolded and stretched. FIG. 2 is a schematic perspective view when the diaper 1 is viewed from the front. FIG. 3A is a schematic cross sectional view taken along a line A-A of FIG. 1, FIG. 3B is a schematic cross sectional view taken along a line B-B of FIG. 1A, and FIG. 3C is a schematic cross sectional view taken along a line C-C of FIG. 1A. Note that "when stretched" in FIG. 1 indicates a state in which a product (diaper 1) is stretched with its wrinkles smoothed. In specific, it indicates a state in which the size of each component constituting the diaper 1 (e.g., a back sheet 30, etc., which will be described later) matches the size of each component alone or the diaper is stretched out up to substantially such a length.

The diaper 1 includes a vertical direction, a lateral direction, and a front and back direction as three directions orthogonal to one another in a pants-shape in FIG. 2. Hereinafter, one side and the other side in the vertical direction in this pants-shaped state are referred to as a "waist opening side" and an "inseam side", respectively, and the front side and the back side in the front and back direction are also referred to as the "front side" and the "back side", respectively.

On the other hand, in the unfolded state in FIG. 1, the diaper 1 includes a longitudinal direction and a width direction as three directions orthogonal to each other. Hereinafter, one side and the other side in the longitudinal direction when unfolded as such are also referred to as a "front side" and a "back side", respectively. Note that the aforementioned width direction when unfolded is the same direction as the aforementioned lateral direction in the pants-shaped state. Accordingly, in the following, the width direction is also referred to as the "lateral direction". Further, the longitudinal direction in such an unfolded state is a direction along the vertical direction in the pants-shaped state. Further, as illustrated in FIG. 3, a direction orthogonal to the vertical direction (longitudinal direction) and the lateral direction (width direction) is referred to as a "thickness direction", and the side to contact a wearer's skin is referred to as a "skin side", and the opposite side is referred to as a "non-skin side".

Further, the diaper 1 includes a front waist portion 3, a back waist portion 4, and a crotch portion 5 in the longitudinal direction, as illustrated in FIG. 1. The front waist portion 3 is a portion positioned on the front side of a wearer when the diaper 1 is worn. The back waist portion 4 is a portion positioned on the back side of the wearer when the diaper 1 is worn. Then, the crotch portion 5 is provided between the front waist portion 3 and the back waist portion 4.

The diaper 1 according to an embodiment of the present disclosure includes an absorbent main body 10, a top sheet 20, a back sheet 30, and leak-proof walls 50. Then, the absorbent main body 10 in the unfolded state of FIG. 1 is folded into two at a predetermined position CL10, serving as a folding position, in the longitudinal direction (vertical direction) of the absorbent main body 10, as well as the front waist portion 3 and the back waist portion 4 opposed to each other in this two-folded state are joined, by welding or the like, at front side edge portions 3es and back side edge portions 4es, respectively. As a result, these waist portions 3, 4 are coupled into an annular shape to form the diaper 1 in the pants-shaped state in which a waist opening BH and a pair of leg openings LH, LH are formed, as illustrated in FIG. 2.

The absorbent main body 10 has a function of absorbing excreta, such as urine, is formed in substantially a rectangular shape in plan view as illustrated in FIG. 1, and is disposed at the center in the lateral direction while the longitudinal direction thereof is directed along the vertical direction of the diaper 1. The absorbent main body 10 includes an absorbent core 11 that is liquid absorbent, and a core-wrapping sheet 12 that covers the outer circumferential face of the core 11. Further, the leak-proof walls 50 are provided on both sides in the lateral direction of the absorbent main body 10. The leak-proof walls 50 will be described later in detail.

The absorbent core 11 is formed such that liquid absorbent fibers such as pulp fibers are formed into a predetermined shape, and has superabsorbent polymer (so-called SAP), or the like mixed thereinside. In an embodiment of the present disclosure, the absorbent core 11 is substantially hour glass shaped in plan view, and has a concave portion 11c in which the central portion in the longitudinal direction of the absorbent core 11 is concave inward in the lateral direction. That is, the absorbent core 11 includes the concave portion 11c, having its dimension in the lateral direction smaller than the dimensions in both end portions 11ea, 11eb in the longitudinal direction of the absorbent core 11, at a position between both the end portions 11ea, 11eb in the longitudinal direction. The core-wrapping sheet 12 is a liquid permeable sheet member that covers the outer circumferential face of the absorbent core 11, and is capable of using a tissue paper, a nonwoven fabric, or the like.

The top sheet 20 is disposed on a skin-side surface in the thickness direction of the absorbent main body 10, and a liquid permeable sheet member to contact a wearer's skin when the diaper 1 is worn. The top sheet 20 according to an embodiment of the present disclosure is formed using, for example, an air-through nonwoven fabric, a spunbond nonwoven fabric, or the like.

The back sheet 30 is disposed on the non-skin side in the thickness direction of the absorbent main body 10, and is a sheet member constituting an outer layer of the diaper 1. In an embodiment of the present disclosure, the back sheet 30 includes a back film 31, an upper outer layer sheet 32 and a lower outer layer sheet 33. The back film 31 is a liquid impermeable and moisture permeable sheet member that is disposed on the non-skin side of the absorbent main body 10, and is formed of, for example, a resin film. The back film 31 is provided, to suppress moisture such as urine absorbed by the absorbent main body 10 from moving to (penetrating) a wearer's clothes .

The upper outer layer sheet 32 and the lower outer layer sheet 33 are flexible sheet-shaped members that are disposed on the non-skin side of the back film 31 while being layered in the thickness direction and, for example, are formed of a spunbond nonwoven fabric or the like. The upper outer layer sheet 32 and the lower outer layer sheet 33 are of the same shape at least in the front waist portion 3 and the back waist portion 4, and have a substantially hourglass shape as in FIG. 1 as a whole. That is, the back sheet 30 includes a concave portion 30c whose central portion (crotch portion 5) in the longitudinal direction is concave, inward in the lateral direction, in a position between the front waist portion 3 and the back waist portion 4.

Further, elastic members such as elastic strings are provided between the upper outer layer sheet 32 and the lower outer layer sheet 33 in the thickness direction. In an embodiment of the present disclosure, as illustrated in FIG. 1, in the front waist portion 3 (a region between the front side edge portions 3es, 3es) of the diaper 1, a plurality of front waist elastic members 35a is joined to and sandwiched between the upper outer layer sheet 32 and the lower outer layer sheet 33, in a state of being stretched in the lateral direction at a predetermined rate of stretch. These front waist elastic members 35a provide stretchability in the lateral direction to the front waist portion 3 of the diaper 1. Similarly, in the back waist portion 4 of the diaper 1 (a region between the back side edge portions 4es, 4es), a plurality of back waist elastic members 35b is joined between the upper outer layer sheet 32 and the lower outer layer sheet 33 in a state of being stretched in the lateral direction at a predetermined rate of stretch. These back waist elastic members 35b provide stretchability in the lateral direction of the back waist portion 4 of the diaper 1.

Note that "the rate of stretch" of the elastic members indicates the degree of stretch assuming that the natural length of the elastic member (elastic string) is 1. For example, when the rate of stretch is 1.2, the elastic member is stretched by 0.2 from the natural strength.

Further, a plurality of back-side leg-circumference elastic members 35lg is provided in the back waist portion 4. The back-side leg-circumference elastic members 35lg are disposed to be curved along the concave portion 30c from a region (a position closer to the center in the longitudinal direction of the absorbent core 11) inside in the longitudinal direction of the back waist portion 4. In specific, the back-side leg-circumference elastic members 35lg each include, as illustrated in FIG. 1, a linear part 35lgs disposed along the lateral direction in a central portion (a region in which the absorbent core 11 is disposed) in the lateral direction, and curved parts 35lgc disposed to be curved obliquely upward toward the outside in the lateral direction from both sides in the lateral direction of the linear part 35lgs. Then, the linear part 35lgs and the curved parts 35lgc are joined between the upper outer layer sheet 32 and the lower outer layer sheet 33 in a state of being stretched at the rates of stretch different from each other.

In an embodiment of the present disclosure, the back-side leg-circumference elastic members 35lg are disposed such that the rate of stretch in the linear part 35lgs is made lower than the rate of stretch in the back waist elastic members 35b. The linear part 35lgs has a portion overlapping the absorbent core 11 (absorbent main body 10) in its central portion in the lateral direction. Thus, reduction in the rate of stretch of the linear part 35lgs can suppress the absorbent core 11 from excessively contracting in the lateral direction. Note that the rate of stretch may vary with the positions in the linear part 35lgs in the lateral direction. For example, the rate of stretch in the central portion in the lateral direction of the linear part 35lgs may be lower than the rate of stretch in both end portions in the lateral direction. Accordingly, a gradient is generated in stress on the linear part 35lgs, thereby being able to restrain contraction of the absorbent core 11 without deteriorating fit.

On the other hand, the curved parts 35lgc are disposed such that the rate of stretch thereof is made higher than the rate of stretch of the back waist elastic members 35b. The curved parts 35lgc are disposed along the concave portion 30c in the back side of the diaper 1. This region is to contact a wearer's buttocks when the diaper 1 is worn, and a portion in which positional deviation is likely to be caused by movement of a wearer's legs. Thus, the rate of stretch in the curved parts 35lgc is increased to strengthen a contractive force, so that positional deviation is not likely to be caused. Further, since the amount of contraction in the back sheet 30 is large in the vicinity of the leg openings LH, the back sheet 30 is easily deformed into a shape along the roundness of a wearer's buttocks, so that fit can be enhanced. Note that, similarly to the linear part 35lgs, the rate of stretch may vary with positions in the lateral direction in the curved parts 35lgc. For example, in the curved parts 35lgc, the rate of stretch in the inside in the lateral direction may be set higher than the rate of stretch in the outside.

### <Leak-proof walls 50>

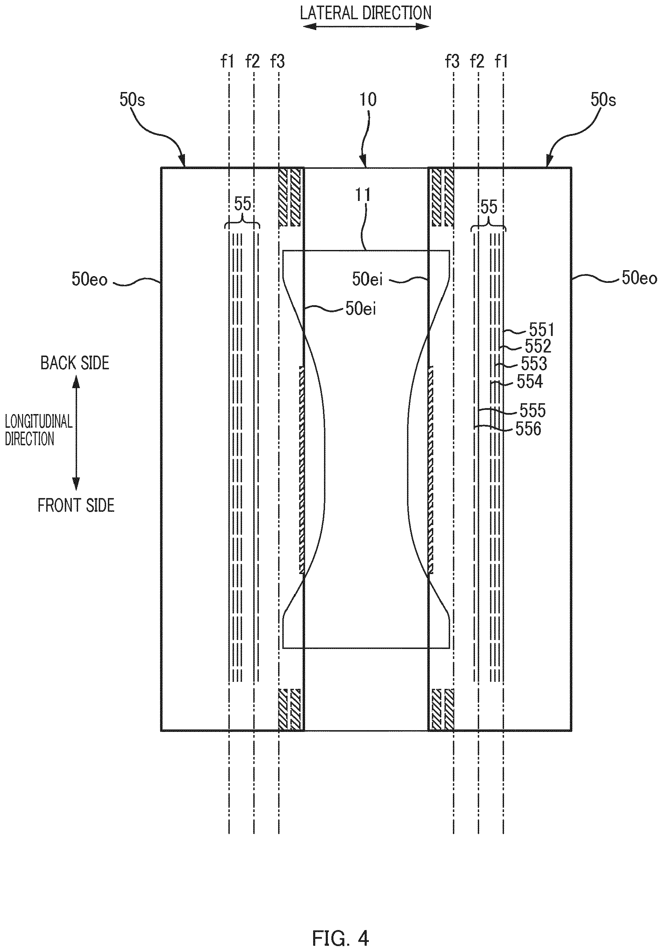

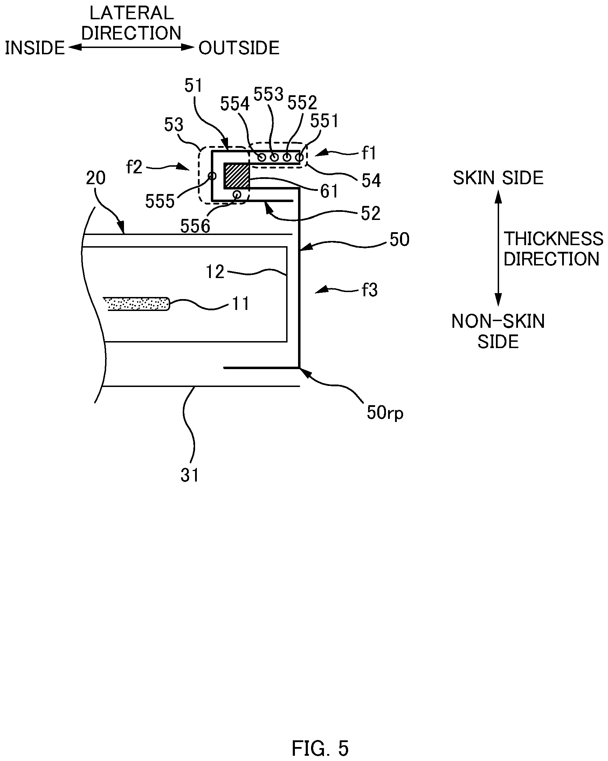

Next, the leak-proof walls 50 will be described. The leak-proof walls 50 are disposed along the longitudinal direction (the vertical direction of the diaper 1) of the absorbent main body 10 on both sides in the lateral direction of the absorbent main body 10, respectively. When the diaper 1 is worn, the leak-proof walls stand up from the side end portions of the absorbent main body 10, to suppress excrement from leaking outside the diaper 1. FIG. 4 is a plan view illustrating the leak-proof walls 50 when unfolded and stretched. FIG. 5 is a schematic enlarged cross sectional view illustrating a region D of FIG. 3C. Note that, in FIG. 5, the scale in the thickness direction is modified for purpose of illustration.

Each of the leak-proof walls 50s is formed such that a rectangular sheet member 50s as illustrated in FIG. 4 is folded in the lateral direction at folding lines f1 to f3 along the longitudinal direction (vertical direction). As the sheet member 50s forming each of the leak-proof walls 50, for example, a sheet member having flexibility such as a nonwoven fabric can be used. Further, in the leak-proof walls 50, a plurality of leak-proof wall elastic members 55 such as elastic strings, which are capable of stretching in the longitudinal direction, are provided. In an example of FIG. 4, six leak-proof wall elastic members 551 to 556, each along the longitudinal direction, are disposed to be aligned from the outside toward the inside in the lateral direction. The leak-proof wall elastic members 551 to 556 each are joined to the leak-proof wall 50 while being stretched in the longitudinal direction at a predetermined rate of stretch. Such leak-proof wall elastic members 55 provided stretchability along the longitudinal direction to the leak-proof walls 50, so as to cause the leak-proof walls 50 to stand up when the diaper 1 is worn.

The leak-proof wall elastic members 55 exhibit stretchability in a range given by dashed lines in FIGS. 1 and 4. Note that the leak-proof wall elastic members 55 that do not exhibit stretchability may exist in a region outside, in the longitudinal direction (vertical direction), the range indicated by dashed lines in FIGS. 1 and 4. Further, the number and arrangement of the leak-proof wall elastic members 55 are not limited to those illustrated in FIG. 4, but may be changed as appropriate according to the use of the diaper 1.

In the unfolded state illustrated in FIG. 4, inner end portions 50ei in the lateral direction of the leak-proof walls 50 (sheet members 50s) are joined/fixed between the absorbent main body 10 and a leak proof film 31 (back sheet 30) in the thickness direction. On the other hand, outer end portions 50eo in the lateral direction of the leak-proof walls 50 (sheet members 50s) extend outside the absorbent main body 10 in the lateral direction. In this state, such a sheet member 50s is folded inward in the lateral direction at the folding line f1 serving as a base point such that the outer end portion 50eo is moved inward, and the sheet member 50s is layered in the thickness direction, while sandwiching the leak-proof wall elastic members 551 to 556 between the layered sheet member 50s, and joined to each other. Note that the outermost elastic member (leak-proof wall elastic members 551 in FIG. 4) in the leak-proof wall elastic members 55 provided in an aligned manner in the lateral direction is disposed to be aligned with the folding line f1 at the position thereof in the lateral direction.

Subsequently, the sheet member 50s is folded to the non-skin side in the thickness direction and inward in the lateral direction at the folding line f2 serving as a base point. The position in the lateral direction of the folding line f2 is substantially the same as the position in the lateral direction of the leak-proof wall elastic member 555. Subsequently, the sheet member 50s is folded to the skin side in the thickness direction and inward in the lateral direction at the folding line f3 serving as a base point. Accordingly, the leak-proof wall 50 folded into substantially an S shape as illustrated in FIGS. 3 and 5 is formed. Note that, in FIG. 5, the position of the folding line f3 is a base part 50rp when the leak-proof wall 50 is raised.

In the leak-proof wall 50, the sheet member 50s is folded back from one side to the other side in the lateral direction, to form a top face portion 51 on the side closest to the skin in the thickness direction. The top face portion 51 is a region having a predetermined width in the lateral direction between the folding line f1 and the folding line f2. In the top face portion 51, the leak-proof wall elastic members 551 to 555 are provided. Further, a lower face portion 52 is formed on the non-skin side in the thickness direction of the top face portion 51. The lower face portion 52 is a region having a predetermined width in the lateral direction between the folding line f2 and the folding line f3. In the lower face portion 52, the leak-proof wall elastic members 555 to 556 are provided. Note that the leak-proof wall elastic member 555 is disposed at substantially the same position in the lateral direction as the folding line f2 as described above, and thus is assumed to be provided in both the top face portion 51 and the lower face portion 52.

Then, in the leak-proof wall 50, a region of the top face portion 51 and a region the lower face portion 52 are joined in the thickness direction through a fold joining part 61. That is, portions of a surface on one side in the thickness direction of the leak-proof wall 50 (sheet member 50s) are joined to each other with the fold joining part 61. Hereinafter, a region inside, in the lateral direction, of a portion in which the fold joining part 61 is formed in the leak-proof wall 50 is also referred to as a fold part 53. In an embodiment of the present disclosure, the fold joining part 61 indicated by a shaded portion in FIG. 5 is provided in a laterally inner region in the top face portion 51 (lower face portion 52), to form the fold part 53. Note that the fold joining part 61 is formed with the use of an adhesive means using an adhesive such as a hot-melt adhesive or a compression boding means such as embossing. The fold joining part 61 is not necessarily formed all over the fold part 53 in the lateral direction as illustrated in FIG. 5. For example, the fold joining part 61 may not be formed at a laterally inner end (position of the folding line f2 in FIG. 5). In a laterally outer region in which the fold joining part 61 is not provided, the top face portion 51 and the lower face portion 52 are not joined to each other, and both of them can be separated in the thickness direction. In the following, a part outside, in the lateral direction, the portion in which the fold joining part 61 is provided in the top face portion 51 is also referred to as a single layer part 54. Note that the wording "single layer" here indicates a layer constituted by only the top face portion 51, and thus the top face portion 51 itself may have a multiple layer structure as in FIG. 5.

In the diaper 1 according to an embodiment of the present disclosure, as illustrated in FIG. 1, the fold joining parts 61 are provided in at least a part of a region corresponding to the concave portion 11c of the absorbent core 11 in the longitudinal direction. Then, the fold joining parts 61 are provided so as not to overlap the absorbent core 11 in the thickness direction.

Such a fold joining part 61 is not provided in regions outside the fold joining part 61 in the longitudinal direction. That is, a region on one end side and a region on the other side in the longitudinal direction with respect to the fold joining part 61 in the leak-proof wall 50 respectively includes non-joining parts 62 where portions of a surface on one side in the thickness direction of the leak-proof wall 50 (sheet member 50s) are not joined to each other. In the non-joining parts 62, as illustrated in FIG. 3B, the top face portion 51 and the lower face portion 52 of the leak-proof wall 50 are not joined to each other. The fold part 53 is not formed, and the entire region in the lateral direction of the top face portion 51 results in a single layer part. Note that, in FIG. 1, the non-joining parts 62 are provided on both sides in the longitudinal direction of the fold joining part 61, respectively, however, the non-joining parts 62 may be provided on only one side in the longitudinal direction.

Further, end joining parts 63 are respectively provided in both end portions in the longitudinal direction of the leak-proof wall 50. In the end joining parts 63, as indicated by shaded portions in FIG. 3A, the top face portion 51 and the lower face portion 52 of the leak-proof wall 50 are joined to each other, as well as the lower face portion 52 and the top sheet 20 are joined to each other. Accordingly, in the end portions in the longitudinal direction of the leak-proof wall 50, the top face portion 51 and the lower face portion 52 are fixed to the skin-side surface of the top sheet 20. Note that, as illustrated in FIG. 1, the end joining parts 63 are disposed on the outside in the lateral direction with respect to the fold joining parts 61. This can restrain outer end portions in the lateral direction in the top face portions 51 from curling up.

### <Effects of leak-proof walls 50>

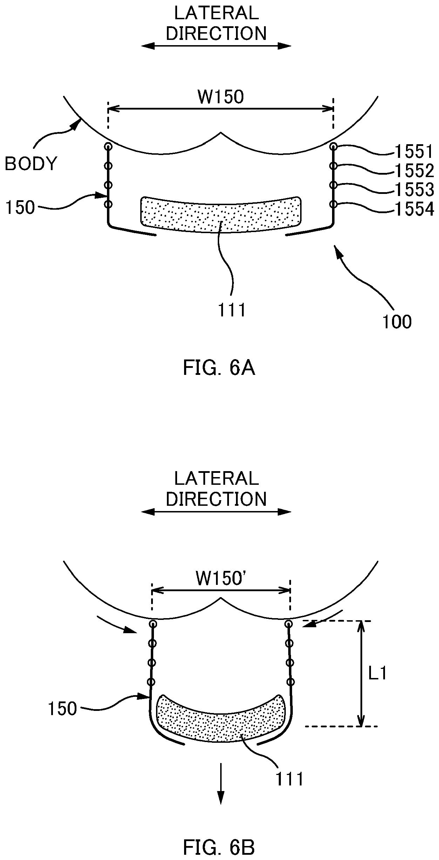

The effects of the leak-proof walls 50 in the diaper 1 will be described. First, a problem caused when wearing a pull-on disposable diaper 100 (hereinafter, also referred to as the "diaper 100") including conventional leak-proof walls (leg gathers) will be described as a comparative example. FIG. 6A is a diagram illustrating a state of wearing the diaper 100 (comparative example) before excretion. FIG. 6B is a diagram illustrating a state of wearing the diaper 100 (comparative example) after excretion.

A basic configuration of the diaper 100 of the comparative example is substantially the same as the diaper 1 according to an embodiment of the present disclosure, however the configuration of the leak-proof wall is greatly different. In the diaper 100 of the comparative example, leak-proof walls 150 in a pair are respectively provided on both end portions in the lateral direction of an absorbent main body 110 (absorbent core 111). In the leak-proof walls 150, a plurality of elastic members 155 such as elastic strings (four elastic members 1551 to 1554 in FIG. 6A) is provided, and the contractive force generated by the elastic members 155 are exerted so that the leak-proof walls 150 stand upward. When the diaper 100 is worn, the leak-proof walls 150 stand up by virtue of the elastic members 1551 to 1554 as illustrated in FIG. 6A, and the contractive force generated by the elastic member 1551 that is positioned at the top part of each of the leak-proof walls 150 is mainly exerted, so that the upper end portions of the leak-proof walls 150 fit along a body of a wearer. At this time, a lateral interval between the pair of leak-proof walls 150 (i.e., a distance in the lateral direction between the tops of the leak-proof walls 150) is referred to as W150.

When excretion is performed in this state, the absorbent core 111 absorbs urine, etc., thereby increasing its weight, and thus is moved so as to droop downward. Then, the leak-proof walls 150 are also pulled due to the absorbent core 111 and deformed so as to be stretched downward as in FIG. 6B. At this time, the height of the standing parts of the leak-proof walls 150 (a distance from the lateral outer end portion of the absorbent core 11 to the position of the top of the leak-proof wall 150) is referred to as L1. Further, the positions of the tops of the leak-proof walls 150 are moved to deviate inward in the lateral direction from the outside, such that a distance W150' between the tops in the lateral direction becomes smaller than the distance W150 between the tops before excretion (W150>W150'). This is because a force to pull the leak-proof walls 150 downward by the absorbent core 111 having an increased weight becomes larger than a contractive force exerted by the elastic member 1551 disposed at the top part of each of the leak-proof walls 150. As a result, it becomes impossible to resist drooping downward of the absorbent core 111 so that the absorbent core 111 greatly droops downward. This may cause a wearer to feel uncomfortable, and/or degradation in fit of the leak-proof walls 150 may bring discomfort to the wearer. Further, when more excretion is added and the weight of the absorbent core 111 increases, the elastic member 1551 disposed at the top part of each of the leak-proof walls 150 is likely to be shifted inward. In specific, on the back side, the position of the elastic member 1551 is likely to deviate further inward with respect to the top of the wearer's buttocks, so that the buttocks are largely exposed outside the diaper 100. This may increase uncomfortableness when wearing the diaper. Further, the tops of the leak-proof walls 150 are disengaged from the wearer's body to create space in leg circumference portions of the diaper 100, so that excrement might leak outside the diaper 100.

It is possible to suppress the positional deviation of the aforementioned leak-proof walls 150 by sufficiently increasing a contractive force exerted by the elastic member 1551. However, in such a case, fitting at the top parts of the leak-proof walls 150 may become too tight such that the leak-proof walls 150 (elastic member 1551) may be caught by a wearer's body, and/or the wearer's skin may be damaged.

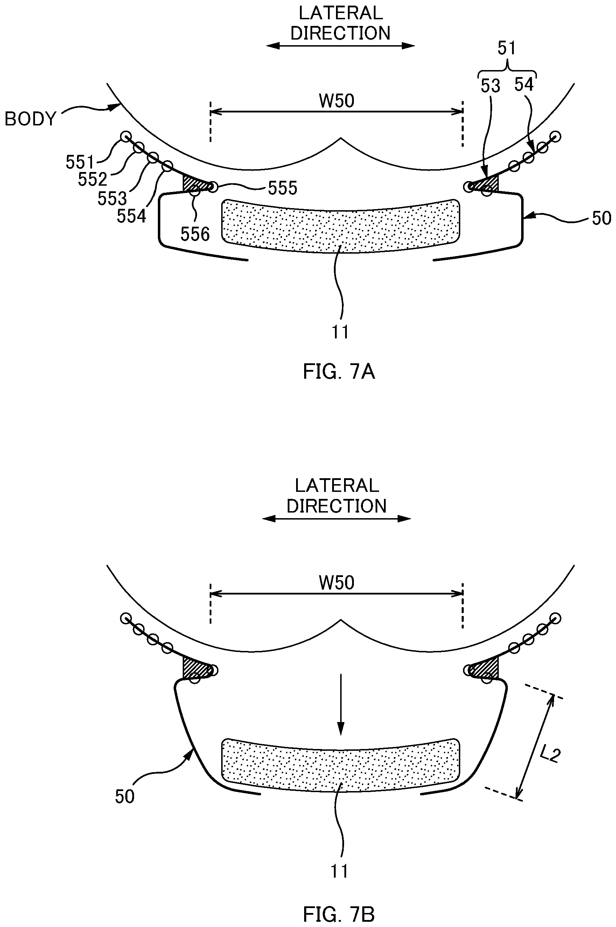

Whereas, in the diaper 1 according to an embodiment of the present disclosure, the leak-proof walls 50 are provided with the aforementioned substantially S-shaped folding structure (see FIG. 5). Thus, even when excretion is performed, it is possible to suppress the creation of a gap in the crotch portion while bringing less discomfort to a wearer. FIG. 7A is a diagram illustrating a state of wearing the diaper 1 before excretion. FIG. 7B is a diagram illustrating a state of wearing the diaper 1 after excretion.

In the state of wearing the diaper 1 illustrated in FIG. 7A, the top face portions 51 disposed on the skin side in the leak-proof walls 50 contact a wearer's body. That is, the top face portions 51 are disposed such that the entire top face portions 51 are in surface contact with the wearer's body. The fold part 53 in such a top face portion 51 is joined in a state where the top face portion 51 and the lower face portion 52 are laminated, thereby increasing stiffness. Thus, when a contractive force exerted onto the fold part 53 by the leak-proof wall elastic members 555 and 556, the fold part 53 is firmly pressed against the wearer's body while maintaining a surface shape. At this time, a lateral interval between the leak-proof walls 50 in a pair (a distance between the fold parts 53, 53) is referred to as W50. On the other hand, the single layer part 54 in the top face portion 51 has stiffness lower than that in the fold part 53 and thus is easily deformable. Thus, when a contractive force is exerted by the leak-proof wall elastic members 551 to 554 that are disposed in the single layer part 54, the single layer part 54 is pressed against the wearer's skin side while forming a curved surface according to a body's projections and depressions.

Accordingly, it becomes possible to closely fit the entire top face portion 51 of each of the leak-proof walls 50 to the wearer's body. Further, since the top face portion 51 is to be in surface contact with the wearer's body, the contractive force exerted by a plurality of leak-proof wall elastic members 551 to 556 is dispersed, so as to increase surface pressure acting onto the wearer's skin as the entire top face portion 51. Thus, as compared with the case where respective contractive forces exerted by the leak-proof wall elastic members 551 to 556 (e.g., elastic strings) are exerted onto the wearer's skin, local pressure increase and deterioration of wear comfort in the diaper 1 are suppressed, and the wearer's skin is less likely to be damaged.

Note that, in an embodiment of the present disclosure, at least one leak-proof wall elastic member 556 is provided to the lower face portion 52 (see FIG. 5). The leak-proof wall elastic member 556 contracts so that the lower face portion 52 and the top face portion 51 are concurrently pressed upward to a wearer's skin side, and thus the fold part 53 easily contacts (comes in surface contact with) a wearer's skin while maintaining a surface shape.

At such a time, it is preferable that the leak-proof wall elastic member 556 is disposed at the same position as the position of the fold joining part 61 in the lateral direction, or at the position inside the fold joining part 61 in the lateral direction. When the leak-proof wall elastic member 556 is disposed outside the fold joining part 61 in the lateral direction, a stretching force of the leak-proof wall elastic member 556 is not exerted substantially on the fold part 53, and thus it is difficult to press the fold part 53 upward to the wearer's skin side while its surface shape being as it is. In contrast, if the leak-proof wall elastic member 556 is disposed below or inside the fold joining part 61, a stretching force by virtue of the leak-proof wall elastic member 556 is exerted in a wide range of the fold part 53 through the fold joining part 61, so that the fold part 53 easily comes in surface contact with the wearer's skin.

When excretion is performed onto the diaper 1 in the state of FIG. 7A, the absorbent core 11 absorbs urine, etc., thereby increasing its weight, and nearly moves to droop downward. However, in the diaper 1 according to an embodiment of the present disclosure, a contractive force of a plurality of elastic members provided to the fold parts 53 and the single layer parts 54 create a force to push the top face portions 51 upward (a wearer's skin side), which serves as a drag against a force to pull the leak-proof walls 50 downward which is caused by an increase in weight of the absorbent core 11. Further, since the top face portions 51 are in surface contact with a wearer's skin, friction on the contact surfaces is large, so that positional deviation of the top face portions 51 are less likely to occur. Accordingly, the lateral interval W50 between the pair of leak-proof walls 50 is less likely to change before and after excretion. As a result, such a state in which the top face portions 51 of the leak-proof walls 50 is in contact with the wearer's skin as in FIG. 7B is easily maintained, and a problem that a space is formed in the leg circumference portions in the diaper 1 is less likely to be caused.

Further, drooping of the absorbent core 11 is more likely to occur, as the standing parts of the leak-proof walls 50 are higher. That is, as illustrated in FIG. 7B, the absorbent core 11 is more easily droop downward, as a distance L2 from the lateral outer end portions of the absorbent core 11 to parts at which the leak-proof walls 50 contact the wearer's skin is longer. Whereas, in the leak-proof wall 50 of the diaper 1, since the fold part 53 is formed, the distance L2 results in being relatively short as compared with a distance L1 of the standing part in each of the leak-proof walls 150 described in FIG. 6B. That is, a part of each of the leak-proof walls 50 is folded over and laminated with the fold part 53, thereby lowering the standing parts of the leak-proof walls 50, so that the absorbent core 11 can be suppressed from greatly drooping downward and discomfort is less likely to be brought to the wearer.

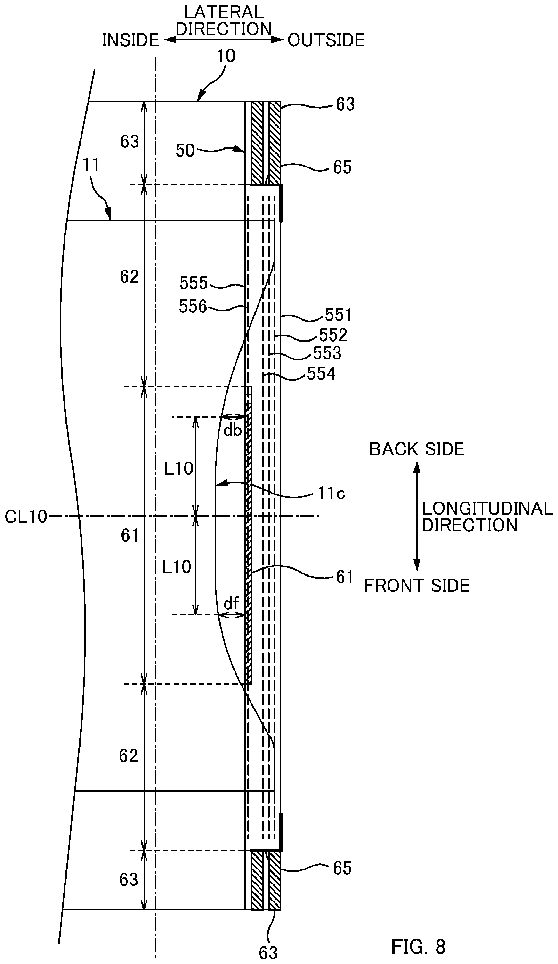

FIG. 8 is a schematic plan view illustrating the arrangement of the fold joining part 61. As illustrated in FIG. 8, the fold joining part 61 (fold part 53) is formed in a region corresponding to the concave portion 11c of the absorbent core 11 in the longitudinal direction. In the region in which the concave portion 11c of the absorbent core 11 is formed, a distance from each of the lateral end portions of the absorbent core 11 to the standing base part 50rp of each of the leak-proof walls 50 is long (see FIG. 5), and accordingly the absorbent core 11 is likely to droop. Thus, the fold joining part 61 is provided in each of the leak-proof walls 50 in this region, to fold over and laminate the leak-proof wall 50 at the fold part 53, so that the distance L2 is shortened as much as possible. This can effectively restrain the absorbent core 11 from drooping.

Furthermore, both ends in the longitudinal direction of the fold joining part 61 (fold part 53) do not reach the concave portion 11c of the absorbent core 11, as in FIG. 8. That is, the fold joining part 61 is formed so as not to overlap the absorbent core 11 in the thickness direction. This is because, in a region direction, a portion having stiffness increased by the fold joining part 61 in each of the leak-proof walls 50 is pressed against the wearer's body (skin side) by the absorbent core 11, so that the texture when the diaper 1 is worn may deteriorate.

With respect to this point, in the diaper 1, the non-joining part 62 is provided outside the leak-proof walls 50 in the longitudinal direction, so that the fold part 53 having high stiffness is not formed in a region in which the non-joining part 62 overlaps the absorbent core 11 in the thickness direction. Further, the region in which the non-joining part 62 is provided is more flexible than a region in which the fold joining part 61 is provided, and thus the leak-proof wall 50 can be easily deformed according to the wearer's body shape. Accordingly, the non-joining parts 62 are provided outside in the longitudinal direction on both sides of each region in which the fold joining part 61 is provided, so that the leak-proof walls 50 easily fit to the wearer's body.

Further, when considering the longitudinal direction of the diaper 1, a point at which the leak-proof wall 50 stands up and contacts the wearer's skin shifts from the leak-proof wall elastic member 555 positioned inside in the lateral direction to the leak-proof wall elastic member 551 positioned outside in the lateral direction, as it goes from the inside to the outside in the longitudinal direction of the diaper 1. That is, a force acts so as to shift the point at which each of the leak-proof walls 50 contacts the wearer's skin to the outside of the top of the wearer's buttocks in the lateral direction and a three-dimensional portion of each of the leak-proof walls 50 is restrained from deviating inside the top of the buttocks in the lateral direction. This can reduce discomfort when the diaper 1 is worn. However, even if the non-joining parts 62 are formed only on one side (e.g., front side) of each of the fold joining parts 61 in the vertical direction, the aforementioned effects can be obtained in this region.

Note that, in the absorbent core 11 according to an embodiment of the present disclosure, the shape of the concave portion 11c is asymmetrical in the longitudinal direction, the depth of the concave portion on the front side is equal to or greater than the depth on the back side. In specific, in FIG. 8, a distance df in the lateral direction between the fold joining part 61 and the concave portion 11c at the position deviating from the center CL10 in the longitudinal direction to the front side by a distance L10 is equal to or greater than a distance db in the lateral direction between the fold joining part 61 and the concave portion 11c at a position deviating from the center CL10 in the longitudinal direction to the back side by a distance L10. As a result, the distance L2 from the outer end portion in the lateral direction of the absorbent core 11 to a portion at which the leak-proof wall 50 contacts a wearer's skin in a back portion (see FIG. 7B) is equal to or smaller than the distance L2 in a front portion. When excrement is discharged while the diaper 1 is worn, the back portion of the absorbent core 11 increases its weight more than that of the front portion and is likely to droop downward. Thus, by making the distance L2 in the back portion shorter than that in the front side, the absorbent core 11 is easily restrained from drooping downward. Further, the distance df is long on the front side, which indicates that the distance in the lateral direction of the absorbent core 11 in this portion becomes narrow. Accordingly, in the movement of putting on the diaper 1, it is easily restrain fingers of a wearer's legs from being caught by the side ends of the absorbent core 11 when the legs are inserted into the leg openings LH.

Further, the end portion in the longitudinal direction in a region in which the stretching force of the leak-proof wall elastic members 55 acts is positioned outside, in the longitudinal direction, the end portion in the longitudinal direction of the fold joining part 61. That is, a stretching force by the leak-proof wall elastic members 55 (555, 556) acts on all the region of the fold joining part 61 (fold part 53). This makes it easier to form the fold part 53 into a flat shape as in FIG. 5 through all the range in the longitudinal direction in which the fold joining part 61 is formed, to easily fit in a flat manner to a wearer's skin. Accordingly, positional deviation of the leak-proof walls 50 is less likely to be caused.

Further, the leak-proof walls 50 are folded back outward (end portion side) from the inside (central side) in the lateral direction of the absorbent main body 10. That is, when being unfolded flat, the leak-proof walls 50 are folded back outward from the inside in the lateral direction so that the top face portions 51 are formed. Then, in a region of the crotch portion 5, the top face portions 51 have free ends on the outer side in the lateral direction. If the top face portions 51 are formed by folding back inward from the outside in the lateral direction to form free ends, fingers of a wearer's legs are likely to be caught by the free ends of the top face portions 51 when the wearer inserts his/her legs into the leg openings LH in putting on the diaper 1. However, in the leak-proof walls 50 according to an embodiment of the present disclosure, the leak-proof walls 50 are formed such that the free ends are positioned on the outer side of the leg openings LH, so that the fingers of the legs are less likely to be caught when putting on the diaper.

Note that the leak-proof walls 50 according to an embodiment of the present disclosure are folded back outward (end portion side) from the inside (central side) in the lateral direction of the absorbent main body 10 also in the front waist portion 3 and the back waist portion 4. That is, in the entire region in the longitudinal direction of the absorbent main body 10 in its unfolded flat state, the leak-proof walls 50 are folded back outward from the inside in the lateral direction to form the top face portions 51, respectively. Since the direction in which the leak-proof walls 50 are folded back are the same in the entire region in the longitudinal direction, the manufacture is easy and the manufacturing costs are lowered. Then, in a predetermined region in the longitudinal direction, the folded and laminated leak-proof walls 50 each are joined, to form the fold joining parts 61, the non-joining parts 62, the end joining parts 63, so that the leak-proof walls 50 formed as in FIGS. 3A to 3C can be formed as appropriate.

Note that, as illustrated in FIG. 3A, in both end portions in the longitudinal direction of each of the leak-proof walls 50, the lower face portion 52 of the leak-proof wall 50 folded back inward in the lateral direction at the folding line f3 is joined to the top sheet 20, in an overlapping state, with the end joining parts 63. Further, the top face portion 51 folded inward in the lateral direction at the folding line f2 is joined to the lower face portion 52, in an overlapping state, with the end joining parts 63. Then, this end joining parts 63 are disposed outside the fold joining part 61 in the lateral direction. Accordingly, in both end portions in the longitudinal direction of the leak-proof walls 50, the leak-proof walls 50 are firmly fixed to the absorbent main body 10 without standing up.

Further, each end joining part 63 is formed in a region outside each fold joining part 61 in the lateral direction, and the lower face portion 52 and the top sheet 20 of each leak-proof wall 50 are not joined to each other, in the inside (on the non-joining part 62 side) in the longitudinal direction with respect to each border part 65 (indicated by solid lines in FIG. 8) including a border part between the end joining part 63 and the non-joining part 62. The lower face portion 52 of each leak-proof wall 50 and the top sheet 20 are joined to each other, in the outside (on the end joining part 63 side) in the longitudinal direction with respect to the border part 65. Accordingly, the border part 65 functions like a pocket so that excrement absorbed by the absorbent core 11 is easily restrained from leaking outside the border part 65 from the absorbent core 11.

Further, as illustrated in FIG. 1, the leak-proof walls 50 are disposed so as to intersect the back-side leg-circumference elastic members 35lg in the thickness direction. In a region on the back side of the pull-on type diaper 1, the leak-proof walls 50 are pulled obliquely upward by a stretching force of the curved parts 35lgc of the back-side leg-circumference elastic members 35lg, so that the leak-proof walls 50 easily stand up when the diaper 1 is worn. Further, the leak-proof walls 50 are pulled, so that the width of the absorbent main body 10 is easily increased. This can enhance fit around the legs when the diaper 1 is worn.

Further, the fold joining parts 61 provided in the leak-proof walls 50 also intersect the back-side leg-circumference elastic members 35lg in the thickness direction. The fold joining parts 61 are pulled obliquely above the diaper 1 by stretching forces of the curved parts 35lgc of the back-side leg-circumference elastic members 35lg, so that the fold parts 53 joined with the fold joining parts 61 further closely contact a wearer's skin, so that space is less likely to be formed in the leg-circumference portions, and fit around the legs when the diaper 1 is worn is improved.

Further, a region in which stretchability of the leak-proof wall elastic members 55 provided in the leak-proof walls 50 acts intersects the front waist elastic members 35a and the back waist elastic members 35b in the thickness direction. Since the leak-proof wall elastic members 55 are supported by stretchability in the lateral direction by the front waist elastic members 35a and the back waist elastic members 35b, the leak-proof walls 50 and the absorbent main body 10 are less likely to droop downward. Accordingly, even if the absorbent core 11 absorbs excrement, etc., thereby increasing its weight, the absorbent main body 10 is restrained from greatly drooping, so that discomfort is less likely to be brought to the wearer.

In the above embodiments, although an example of using elastic strings as a waist elastic member and a leak-proof wall elastic member is described, these elastic members are not limited to linear elastic members such as so-called elastic string. For example, a planar (band shaped) elastic member having a predetermined width may be used. Further, a sheet member constituting a leak-proof wall and a back sheet may be a sheet member having stretchability (e.g., stretchable nonwoven fabric), so that a configuration may be such that an elastic member such as an elastic string is separately provided.

### [Reference signs list]

1 diaper (pull-on disposable diaper),
3 front waist portion, 3es front side edge portion,
4 back waist portion, 4es back side edge portion,
5 crotch portion ,
10 absorbent main body,
11 absorbent core, 11c concave portion, 11ea end portion,
11eb end portion,
12 core-wrapping sheet,
20 top sheet,
30 back sheet , 30c concave portion,
31 back film, 32 upper outer layer sheet, 33 lower outer layer sheet,
35a front waist elastic member, 35b back waist elastic member,
35lg back-side leg-circumference elastic member, 35lgs linear part, 35lgc curved part,
50 leak-proof wall,
50s sheet member, 50ei inner end portion, 50eo outer end portion, 50rp base part,
51 top face portion, 52 lower face portion, 53 fold part, 54 single layer part,
55 leak-proof wall elastic member, 551 to 556 leak-proof wall elastic member,
61 fold joining part, 62 non-joining part, 63 end joining part,
65 boarder part,
100 diaper (comparative example),
110 absorbent main body, 111 absorbent core,
150 leak-proof wall,
155 the elastic members 1551 to 1554 elastic member,
f1,f2,f3 folding line,
BH waist opening, LH leg opening,
CL10 central portion,
df distance, db distance, L1 the distance L2 distance,
W50 interval, W150 distance, W150' distance

## Claims

1. An absorbent article (1), comprising:
an absorbent main body (10) including an absorbent core (11) having a longitudinal direction and a lateral direction intersecting each other; and
leak-proof walls (50) in a pair respectively provided on both sides in the lateral direction of the absorbent main body, the leak-proof walls including elastic members (55) that are stretchable in the longitudinal direction,
the absorbent core (11) including a concave portion (11c) that is concave inward in the lateral direction in the central portion in the longitudinal direction,
a dimension in the lateral direction of the concave portion (11c) provided in the absorbent core being smaller than a dimension in the lateral direction of both ends (11ea, 11eb) in the longitudinal direction of the absorbent core,
the leak-proof walls (50) each including a joining part (61) where portions of a surface of each of the leak-proof walls are joined to each other on one side in a thickness direction,
the leak-proof walls each including a non-joining part (62) in at least one of a region on one end side and a region on another end side in the longitudinal direction with respect to the joining part (61), the non-joining part (62) being where portions of a surface of each of the leak-proof walls (50) are not joined to each other on the one side in a thickness direction,
the joining part (61) provided in each of the leak-proof walls in a pair not overlapping the absorbent core (11) in the thickness direction,
a distance (W50) between the joining parts (61) provided to the leak-proof walls (50) in a pair being greater in the lateral direction than a minimum dimension of the concave portion (11c) and smaller in the lateral direction than a dimension of both the ends (11ea, 11eb) in the longitudinal direction of the absorbent core.

2. An absorbent article according to claim 1, wherein
the non-joining part (62) is included in each of a region on one end side and a region on the other end side in the longitudinal direction with respect to the joining part (61) in each of the leak-proof walls (50).

3. An absorbent article according to claim 1 or 2, wherein
an end in the longitudinal direction of a region where a stretching force of the elastic member (55) acts is positioned outside in the longitudinal direction with respect to an end in the longitudinal direction of the joining part (61).

4. An absorbent article according to any one of claims 1 to 3, wherein
the leak-proof walls (50) each include a portion obtained by folding each of the leak-proof walls outward from inside in the lateral direction at a folding line along the longitudinal direction.

5. An absorbent article according to claim 4, wherein
the portion obtained by folding each of the leak-proof walls outward from inside in the lateral direction includes
a fold part (53) obtained by joining folded portions of the leak-proof wall to each other with the joining part (61), and
a single layer part (54) obtained by not joining folded portions of the leak-proof wall, in an outside of the fold part in the lateral direction, and
stiffness, of the leak-proof wall, in the fold part (53) is greater than stiffness, of the leak-proof wall, in the single layer part (54).

6. An absorbent article according to claim 5, wherein
the fold part (53) includes
a top face portion (51) to contact a wearer's skin, and
a lower face portion (52) overlapping the top face portion on its lower side.

7. An absorbent article according to claim 6, wherein
a position of the elastic member (556) that is provided to the lower face portion (52) is same as or inside, in the lateral direction, a position of the joining part (61).

8. An absorbent article according to any one of claims 4 to 7, wherein
a top sheet (20) is provided on a skin side of the absorbent body (10),
the leak-proof walls (50) each include
a lower face portion (52) obtained by folding the leak-proof wall (50) inward from outside in the lateral direction to overlap the top sheet (20) on its skin side, and
a top face portion (51) obtained by folding the leak-proof wall (50) outward from inside in the lateral direction to overlap the lower face portion (52) on its skin side, and
the top sheet (20) and the lower face portion (52) are joined in a region outside the non-joining part (62) in the longitudinal direction as well as outside the joining part (61) in the lateral direction, so that the lower face portion (52) and the top face portion (51) are joined to each other.

9. An absorbent article according to any one of claims 1 to 8, wherein
a distance (df) in the lateral direction between the joining part (61) and the concave portion (11c) in a position deviating from a center (CL10) in the longitudinal direction of the absorbent core by a predetermined distance (L10) is equal to or greater than a distance (db) in the lateral direction between the joining part (61) and the concave portion (11c) at a position deviating from a center (CL10) in the longitudinal direction of the absorbent core (11) to a back side by the predetermined distance (L10).

10. An absorbent article according to any one of claims 1 to 9, wherein
a leg-circumference elastic member (351g) configured to stretch in the lateral direction is disposed on the back side in the longitudinal direction,
the leg-circumference elastic member includes
a linear part (351gs) disposed along the lateral direction so as to overlap the absorbent core (11) in the thickness direction in a central portion in the lateral direction, and
curved parts (351gc) disposed to be curved from both sides in the lateral direction of the linear part to outside in the lateral direction as well as to the back side in the longitudinal direction, and
the leak-proof wall (50) and the leg-circumference elastic member (351g) have a portion intersecting each other in the thickness direction.

11. An absorbent article according to claim 10, wherein
the joining part (61) and the leg-circumference elastic member (351g) have a portion intersecting each other in the thickness direction.

12. An absorbent article according to claim 10 or 11, wherein
the rate of stretch in the linear part is greater than the rate of stretch in the curved parts (351gc) in the leg-circumference elastic member.

13. An absorbent article according to any one of claims 1 to 12, comprising:
a front waist elastic member (35a) is disposed along the lateral direction on the front side in the longitudinal direction, the front waist elastic member being stretchable in the lateral direction; and
a back waist elastic member (35b) is disposed along the lateral direction on the back side in the longitudinal direction, the back waist elastic member being stretchable in the lateral direction, and
at least one of a region where stretchability is exerted by the front waist elastic member (35a) and a region where stretchability is exerted by the back waist elastic member (35b) intersects, in the thickness direction, a region where stretchability is exerted by the elastic members (55) provided to the leak-proof walls (50).

## Patentansprüche

1. Absorbierender Artikel (1), der Folgendes umfasst:
einen absorbierenden Hauptkörper (10), der einen Saugkern (11) einschließt, der eine Längsrichtung und eine laterale Richtung aufweist, die einander schneiden; und
auslaufsichere Wände (50) im Paar, die jeweils auf beiden Seiten in der lateralen Richtung des absorbierenden Hauptkörpers bereitgestellt sind, wobei die auslaufsicheren Wände elastische Elemente (55) einschließen, die in der Längsrichtung dehnbar sind,
wobei der Saugkern (11) einen konkaven Abschnitt (11c) einschließt, der nach innen in der lateralen Richtung in dem mittleren Abschnitt in der Längsrichtung konkav ist,
eine Abmessung in der lateralen Richtung des konkaven Abschnitts (11c), der in dem Saugkern bereitgestellt ist, kleiner ist als eine Abmessung in der lateralen Richtung von beiden Enden (11ea, 11eb) in der Längsrichtung des Saugkerns,
die auslaufsicheren Wände (50) jeweils ein Verbindungsteil (61) einschließen, wo Abschnitte einer Oberfläche von jeder der auslaufsicheren Wände miteinander auf einer Seite in der Dickenrichtung verbunden sind,
die auslaufsicheren Wände jeweils ein Nicht-Verbindungsteil (62) in mindestens einem von einem Bereich auf einer Endseite und einem Bereich auf einer anderen Endseite in der Längsrichtung, in Bezug auf das Verbindungsteil (61), einschließen, wobei das Nicht-Verbindungsteil (62) vorliegt, wo Abschnitte einer Oberfläche von jeder der auslaufsicheren Wände (50) nicht miteinander auf der einen Seite in der Dickenrichtung verbunden sind,
das Verbindungsteil (61), das in jeder der auslaufsicheren Wände im Paar bereitgestellt ist, nicht mit dem Saugkern (11) in der Dickenrichtung überlappt,
ein Abstand (W50) zwischen den Verbindungsteilen (61), die für die auslaufsicheren Wände (50) im Paar bereitgestellt sind, in der lateralen Richtung größer ist als eine minimale Abmessung des konkaven Abschnitts (11c) und in der lateralen Richtung kleiner ist als eine Abmessung von beiden der Enden (11ea, 11eb) in der Längsrichtung des Saugkerns.

2. Absorbierender Artikel nach Anspruch 1, wobei
das Nicht-Verbindungsteil (62) in jedem von einem Bereich auf einer Endseite und einem Bereich auf der anderen Endseite in der Längsrichtung, in Bezug auf das Verbindungsteil (61), in jeder der auslaufsicheren Wände (50) eingeschlossen ist.

3. Absorbierender Artikel nach Anspruch 1 oder 2, wobei
ein Ende in der Längsrichtung eines Bereichs, wo eine Dehnkraft des elastischen Elements (55) wirkt, außerhalb in der Längsrichtung, in Bezug auf ein Ende in der Längsrichtung des Verbindungsteils (61), positioniert ist.

4. Absorbierender Artikel nach einem der Ansprüche 1 bis 3, wobei
die auslaufsicheren Wände (50) jeweils einen Abschnitt einschließen, der durch Falten von jeder der auslaufsicheren Wände nach außen von innen in der lateralen Richtung an einer Faltlinie entlang der Längsrichtung erhalten wird.

5. Absorbierender Artikel nach Anspruch 4, wobei
der Abschnitt, der durch Falten von jeder der auslaufsicheren Wände nach außen von innen in der lateralen Richtung erhalten wird, Folgendes einschließt:
ein Faltteil (53), das durch Verbinden von gefalteten Abschnitten der auslaufsicheren Wand miteinander mit dem Verbindungsteil (61) erhalten wird, und
ein Einzelschichtteil (54), das durch Nicht-Verbinden von gefalteten Abschnitten der auslaufsicheren Wand erhalten wird, in einer Außenseite des Faltteils in der lateralen Richtung, und
die Steifigkeit der auslaufsicheren Wand in dem Faltteil (53) größer ist als die Steifigkeit der auslaufsicheren Wand in dem Einzelschichtteil (54).

6. Absorbierender Artikel nach Anspruch 5, wobei
das Faltteil (53) Folgendes einschließt:
einen Oberseitenabschnitt (51) zum Kontakt mit der Haut eines Trägers und
einen Unterseitenabschnitt (52), der mit dem Oberseitenabschnitt auf seiner unteren Seite überlappt.

7. Absorbierender Artikel nach Anspruch 6, wobei
eine Position des elastischen Elements (556), das für den Unterseitenabschnitt (52) bereitgestellt ist, in der lateralen Richtung gleich oder innerhalb einer Position des Verbindungsteils (61) ist.

8. Absorbierender Artikel nach einem der Ansprüche 4 bis 7, wobei
eine obere Lage (20) auf einer Hautseite des Saugkörpers (10) bereitgestellt ist,
die auslaufsicheren Wände (50) jeweils Folgendes einschließen:
einen Unterseitenabschnitt (52), der durch Falten der auslaufsicheren Wand (50) nach innen von außen in der lateralen Richtung erhalten wird, um mit der oberen Lage (20) auf ihrer Hautseite zu überlappen, und
einen Oberseitenabschnitt (51), der durch Falten der auslaufsicheren Wand (50) nach außen von innen in der lateralen Richtung erhalten wird, um mit dem Unterseitenabschnitt (52) auf seiner Hautseite zu überlappen, und
die obere Lage (20) und der Unterseitenabschnitt (52) in einem Bereich außerhalb des Nicht-Verbindungsteils (62) in der Längsrichtung sowie außerhalb des Verbindungsteils (61) in der lateralen Richtung verbunden sind, sodass der Unterseitenabschnitt (52) und der Oberseitenabschnitt (51) miteinander verbunden sind.

9. Absorbierender Artikel nach einem der Ansprüche 1 bis 8, wobei
ein Abstand (df) in der lateralen Richtung zwischen dem Verbindungsteil (61) und dem konkaven Abschnitt (11c) in einer Position, die von einer Mitte (CL10) in der Längsrichtung des Saugkörpers um einen vorgegebenen Abstand (L10) abweicht, gleich oder größer ist als ein Abstand (db) in der lateralen Richtung zwischen dem Verbindungsteil (61) und dem konkaven Abschnitt (11c) an einer Position, die von einer Mitte (CL10) in der Längsrichtung des Saugkörpers (11) zu einer hinteren Seite um den vorgegebenen Abstand (L10) abweicht.

10. Absorbierender Artikel nach einem der Ansprüche 1 bis 9, wobei
ein elastisches Element des Beinumfangs (351g), das zur Dehnung in der lateralen Richtung konfiguriert ist, auf der hinteren Seite in der Längsrichtung angeordnet ist,
das elastische Element des Beinumfangs Folgendes einschließt:
ein lineares Teil (351gs), das entlang der lateralen Richtung angeordnet ist, um mit dem Saugkern (11) in der Dickenrichtung in einem mittleren Abschnitt in der lateralen Richtung zu überlappen, und
gekrümmte Teile (351gc), die zur Krümmung von beiden Seiten der lateralen Richtung des linearen Teils nach außen in der lateralen Richtung sowie zu der hinteren Seite in der Längsrichtung angeordnet sind, und
die auslaufsichere Wand (50) und das elastische Element des Beinumfangs (351g) einen Abschnitt aufweisen, in dem sie sich miteinander in der Dickenrichtung schneiden.

11. Absorbierender Artikel nach Anspruch 10, wobei
das Verbindungsteil (61) und das elastische Element des Beinumfangs (351g) einen Abschnitt aufweisen, in dem sie sich miteinander in der Dickenrichtung schneiden.

12. Absorbierender Artikel nach Anspruch 10 oder 11, wobei
die Dehnungsrate in dem linearen Teil größer ist als die Dehnungsrate in den gekrümmten Teilen (351gc) in dem elastischen Element des Beinumfangs.

13. Absorbierender Artikel nach einem der Ansprüche 1 bis 12, der Folgendes umfasst:
ein elastisches Element der vorderen Taille (35a), das entlang der lateralen Richtung auf der vorderen Seite in der Längsrichtung angeordnet ist, wobei das elastische Element der vorderen Taille in der lateralen Richtung dehnbar ist; und
ein elastisches Element der hinteren Taille (35b), das entlang der lateralen Richtung auf der hinteren Seite in der Längsrichtung angeordnet ist, wobei das elastische Element der hinteren Taille in der lateralen Richtung dehnbar ist, und
mindestens einen von einem Bereich, wo die Dehnbarkeit durch das elastische Element der vorderen Taille (35a) ausgeübt wird, und einem Bereich, wo die Dehnbarkeit durch das elastische Element der hinteren Taille (35b) ausgeübt wird, der sich in der Dickenrichtung mit einem Bereich schneidet, wo die Dehnbarkeit durch die elastischen Elemente (55) ausgeübt wird, die für die auslaufsicheren Wände (50) bereitgestellt sind.

## Revendications

1. Article absorbant (1), comportant :
un corps principal absorbant (10) comprenant une partie centrale absorbante (11) ayant une direction allant dans le sens longitudinal et une direction allant dans le sens latéral se croisant l'une par rapport à l'autre ; et
des parois antifuites (50), formées en une paire, qui sont mises respectivement en œuvre des deux côtés dans la direction allant dans le sens latéral du corps principal absorbant, les parois antifuites comprenant des éléments élastiques (55) qui sont en mesure de s'étirer dans la direction allant dans le sens longitudinal,
la partie centrale absorbante (11) comprenant une partie concave (11c) qui est concave vers l'intérieur dans la direction allant dans le sens latéral dans la partie du centre dans la direction allant dans le sens longitudinal,
une dimension dans la direction allant dans le sens latéral de la partie concave (11c) mise en œuvre dans la partie centrale absorbante étant inférieure par rapport à une dimension dans la direction allant dans le sens latéral des deux extrémités (11ea, 11eb) dans la direction allant dans le sens longitudinal de la partie centrale absorbante,
les parois antifuites (50) comprenant chacune une partie d'assemblage (61) où des parties d'une surface de chacune des parois antifuites sont assemblées l'une par rapport à l'autre sur un côté dans une direction allant dans le sens de l'épaisseur,
les parois antifuites comprenant chacune une partie de non-assemblage (62) dans au moins l'une parmi une région sur un côté d'extrémité et une région sur un autre côté d'extrémité dans la direction allant dans le sens longitudinal par rapport à la partie d'assemblage (61), la partie de non-assemblage (62) étant là où des parties d'une surface de chacune des parois antifuites (50) ne sont pas assemblées l'une par rapport à l'autre sur ledit un côté dans une direction allant dans le sens de l'épaisseur,
la partie d'assemblage (61) étant mise en œuvre dans chacune des parois antifuites, formées en une paire, ne chevauchant pas la partie centrale absorbante (11) dans la direction allant dans le sens de l'épaisseur,
une distance (W50) entre les parties d'assemblage (61) mises en œuvre au niveau des parois antifuites (50), formées en une paire, étant supérieure dans la direction allant dans le sens latéral par rapport à une dimension minimum de la partie concave (11c) et inférieure dans la direction allant dans le sens latéral par rapport à une dimension des deux extrémités (11ea, 11eb) dans la direction allant dans le sens longitudinal de la partie centrale absorbante.

2. Article absorbant selon la revendication 1, dans lequel
la partie de non-assemblage (62) est incluse dans chacune parmi une région sur un côté d'extrémité et une région sur l'autre côté d'extrémité dans la direction allant dans le sens longitudinal par rapport à la partie d'assemblage (61) dans chacune des parois antifuites (50).

3. Article absorbant selon la revendication 1 ou la revendication 2, dans lequel
une extrémité dans la direction allant dans le sens longitudinal d'une région où une force d'étirage de l'élément élastique (55) agit est positionnée à l'extérieur dans la direction allant dans le sens longitudinal par rapport à une extrémité dans la direction allant dans le sens longitudinal de la partie d'assemblage (61).

4. Article absorbant selon l'une quelconque des revendications 1 à 3, dans lequel
les parois antifuites (50) comprennent chacune une partie obtenue en pliant chacune des parois antifuites vers l'extérieur depuis l'intérieur dans la direction allant dans le sens latéral au niveau d'une ligne de pliage le long de la direction allant dans le sens longitudinal.

5. Article absorbant selon la revendication 4, dans lequel
la partie obtenue en pliant chacune des parois antifuites vers l'extérieur depuis l'intérieur dans la direction allant dans le sens latéral comprend
une partie formant pli (53) obtenue en assemblant les parties pliées de la paroi antifuite l'une par rapport à l'autre avec la partie d'assemblage (61), et
une partie à une seule couche (54) obtenue en n'assemblant pas les parties pliées de la paroi antifuite, dans une partie extérieure de la partie formant pli dans la direction allant dans le sens latéral, et
la rigidité, de la paroi antifuite, dans la partie formant pli (53) est supérieure par rapport à la rigidité, de la paroi antifuite, dans la partie à une seule couche (54).

6. Article absorbant selon la revendication 5, dans lequel
la partie formant pli (53) comprend
une partie formant face supérieure (51) destinée à entrer en contact avec la peau d'un utilisateur, et
une partie formant face inférieure (52) chevauchant la partie formant face supérieure sur son côté inférieur.

7. Article absorbant selon la revendication 6, dans lequel
une position de l'élément élastique (556) qui est mis en œuvre au niveau de la partie formant face inférieure (52) est identique ou à l'intérieur, dans la direction allant dans le sens latéral, par rapport à une position de la partie d'assemblage (61).

8. Article absorbant selon l'une quelconque des revendications 4 à 7, dans lequel
une feuille de dessus (20) est mise en œuvre sur un côté peau du corps absorbant (10),
les parois antifuites (50) comprennent chacune
une partie formant face inférieure (52) obtenue en pliant la paroi antifuite (50) vers l'intérieur depuis l'extérieur dans la direction allant dans le sens latéral pour chevaucher la feuille de dessus (20) sur son côté peau, et
une partie formant face supérieure (51) obtenue en pliant la paroi antifuite (50) vers l'extérieur depuis l'intérieur dans la direction allant dans le sens latéral pour chevaucher la partie formant face inférieure (52) sur son côté peau, et
la feuille de dessus (20) et la partie formant face inférieure (52) sont assemblées dans une région à l'extérieur de la partie de non-assemblage (62) dans la direction allant dans le sens longitudinal ainsi qu'à l'extérieur de la partie d'assemblage (61) dans la direction allant dans le sens latéral, de telle sorte que la partie formant face inférieure (52) et la partie formant face supérieure (51) sont assemblées l'une par rapport à l'autre.

9. Article absorbant selon l'une quelconque des revendications 1 à 8, dans lequel
une distance (df) dans la direction allant dans le sens latéral entre la partie d'assemblage (61) et la partie concave (11c) dans une position déviant d'un centre (CL10) dans la direction allant dans le sens longitudinal de la partie centrale absorbante selon une distance prédéterminée (L10) est égale ou supérieure par rapport à une distance (db) dans la direction allant dans le sens latéral entre la partie d'assemblage (61) et la partie concave (11c) au niveau d'une position déviant d'un centre (CL10) dans la direction allant dans le sens longitudinal de la partie centrale absorbante (11) jusqu'à un côté arrière selon la distance prédéterminée (L10).

10. Article absorbant selon l'une quelconque des revendications 1 à 9, dans lequel
un élément élastique au niveau de la circonférence de la jambe (351g) configuré pour s'étirer dans la direction allant dans le sens latéral est disposé sur le côté arrière dans la direction allant dans le sens longitudinal,
l'élément élastique au niveau de la circonférence de la jambe comprend
une partie linéaire (351gs) disposée le long de la direction allant dans le sens latéral de manière à chevaucher la partie centrale absorbante (11) dans la direction allant dans le sens de l'épaisseur dans une partie du centre dans la direction allant dans le sens latéral, et
des parties courbes (351gc) disposées pour être courbées en provenance des deux côtés dans la direction allant dans le sens latéral de la partie linéaire jusqu'à l'extérieur dans la direction allant dans le sens latéral ainsi que jusqu'au côté arrière dans la direction allant dans le sens longitudinal, et
la paroi antifuite (50) et l'élément élastique au niveau de la circonférence de la jambe (351g) ont une partie se croisant l'une par rapport à l'autre dans la direction allant dans le sens de l'épaisseur.

11. Article absorbant selon la revendication 10, dans lequel
la partie d'assemblage (61) et l'élément élastique au niveau de la circonférence de la jambe (351g) ont une partie se croisant l'une par rapport à l'autre dans la direction allant dans le sens de l'épaisseur.

12. Article absorbant selon la revendication 10 ou la revendication 11, dans lequel
le taux d'étirage dans la partie linéaire est supérieur par rapport au taux d'étirage dans les parties courbes (351gc) dans l'élément élastique au niveau de la circonférence de la jambe.

13. Article absorbant selon l'une quelconque des revendications 1 à 12, comportant :
un élément élastique avant au niveau de la taille (35a) qui est disposé le long de la direction allant dans le sens latéral sur le côté avant dans la direction allant dans le sens longitudinal, l'élément élastique avant au niveau de la taille étant en mesure de s'étirer dans la direction allant dans le sens latéral ; et
un élément élastique arrière au niveau de la taille (35b) qui est disposé le long de la direction allant dans le sens latéral sur le côté arrière dans la direction allant dans le sens longitudinal, l'élément élastique arrière au niveau de la taille étant en mesure de s'étirer dans la direction allant dans le sens latéral, et
au moins l'une parmi une région où l'étirage est exercé par l'élément élastique avant au niveau de la taille (35a) et une région où l'étirage est exercé par l'élément élastique arrière au niveau de la taille (35b) qui croise, dans la direction allant dans le sens de l'épaisseur, une région où l'étirage est exercé par les éléments élastiques (55) mis en œuvre au niveau des parois antifuites (50).
